(19) **Europäisches Patentamt / European Patent Office / Office européen des brevets**

(11) **EP 2 301 976 A1**

(12) **EUROPEAN PATENT APPLICATION**
published in accordance with Art. 153(4) EPC

(43) Date of publication:
**30.03.2011 Bulletin 2011/13**

(21) Application number: **09766534.3**

(22) Date of filing: **05.06.2009**

(51) Int Cl.:
*C08F 20/06* (2006.01)　　*A61F 7/02* (2006.01)
*A61F 7/10* (2006.01)　　*A61K 8/02* (2006.01)
*A61K 8/81* (2006.01)　　*A61K 9/70* (2006.01)
*A61K 47/02* (2006.01)　　*A61K 47/10* (2006.01)
*A61K 47/32* (2006.01)　　*A61Q 19/00* (2006.01)
*C09K 5/08* (2006.01)

(86) International application number:
**PCT/JP2009/060330**

(87) International publication number:
**WO 2009/154086 (23.12.2009 Gazette 2009/52)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **17.06.2008 JP 2008158081**

(71) Applicant: **Sumitomo Seika Chemicals CO. LTD.**
**Kako-gun**
**Hyogo 675-0145 (JP)**

(72) Inventors:
• **MIURA, Kazuyuki**
**Himeji-shi**
**Hyogo 672-8076 (JP)**
• **KOBAYASHI, Shinji**
**Kako-gun**
**Hyogo 675-0145 (JP)**

(74) Representative: **HOFFMANN EITLE**
**Patent- und Rechtsanwälte**
**Arabellastraße 4**
**81925 München (DE)**

(54) **WATER-SOLUBLE ACRYLIC ACID SALT POLYMER AND GELLING BASE**

(57) An object of the present invention is to provide a water-soluble acrylate polymer that can form a gelling base. When used in forming a poultice or cooling sheet, the gelling base not only imparts desirable stretchability, but also has an appropriate reaction rate with a polyvalent metal. The gelling base is free from gel penetration to the back side of a support or gel seeping from the support while the gel is being cured, and is easily applied to the support. When the gelling base is gelated by being allowed to stand under the conditions of 25°C and a relative humidity of 60%, the gel strength is 3,200 to 7,000 dyn/cm$^2$ after standing for 7.5 hours and 5,000 to 9,000 dyn/cm$^2$ after standing for 200 hours, and satisfies the following relationship: Gel strength after standing for 7.5 hours] ≤ Gel strength after standing for 200 hours.

**EP 2 301 976 A1**

**Description**

TECHNICAL FIELD

The present invention relates to a water-soluble acrylate polymer, a gelling base containing the water-soluble acrylate polymer, and a poultice and cooling sheet containing the gelling base.

BACKGROUND ART

In poultices or cooling sheets, which are formed by applying a mixture of medicament, water and other ingredients to the surface of a nonwoven fabric or like support, a gelling base comprising a water-soluble polymer of (meth) acrylic acid or salt thereof is often used.

Such a gelling base is required to have sufficient adhesiveness to the skin, stretchability, which is necessary for application to a bendable part of the body, and other characteristics.

In addition to the aforementioned characteristics, various other characteristics are required to facilitate the production process. A typical process for producing a poultice or cooling sheet comprises the steps of applying a gelling base composed of various ingredients to a nonwoven fabric or like support, covering the surface thereof with a polyethylene film or like liner, cutting, packing, and curing and aging the gel in the package.

Aluminum and other polyvalent metals are used for curing the gel. If the reaction between the gel and the polyvalent metal proceeds undesirably slow (i.e., if the curing speed is too slow), gel penetration to the back side of the support, gel seeping from the support, and other problems arise. If the reaction between the gel and the polyvalent metal proceeds too quickly (i.e., if the curing speed is too fast), the resulting gel becomes too hard; making the application of the gel to the support difficult. Accordingly, the gelling base must have properties that enable it to react with the polyvalent metal at a desirable rate.

A poultice base is proposed in Patent Document 1 as one example of a gelling base that has the aforementioned characteristics. The poultice base is made of a copolymer comprising (meth)acrylic acid and a salt thereof as its main construction monomers, wherein the copolymer has an acid to salt molar ratio of 40/60 to 70/30, and a viscosity of 200 to 450 mPa·s at 20°C in the form of a 0.2 mass% aqueous solution. However, this gelling base does not have a suitable reaction rate with a polyvalent metal. This causes problems such as the gel penetrating to the back side of a support and seeping from the support while the gel is being cured. Furthermore, the resulting gel becomes too hard, making it difficult to apply it to the support.

In view of the problems described above, the development of a gelling base that reacts with a polyvalent metal at a desirable rate is desired.

Prior Art Document

Patent Document

Patent Document 1: Japanese Unexamined Patent Publication No. 2007-320939

DISCLOSURE OF THE INVENTION

Problem to Be Solved by the Invention

An object of the present invention is to provide a gelling base that has desirable stretchability for use in a poultice or cooling sheet, exhibits an appropriate reaction rate with a polyvalent metal, that is free from gel penetration to the back side of a support and gel seeping from the support while the gel is being cured, and that is easily applied to a support. Another object of the present invention is to provide a water-soluble acrylate polymer contained in the gelling base.

Means for Solving the Problem

The present invention provides the water-soluble acrylate polymers, gelling base, poultice and cooling sheet described below.

Item 1. A water-soluble acrylate polymer that makes it possible to obtain a gelling base having a gel strength recited below when the gelling base comprises the components and the proportions thereof listed below;
when the gelling base is gelated by being allowed to stand under the conditions of 25°C and a relative humidity of 60%, the resulting gel has a gel strength of 3,200 to 7,000 $dyn/cm^2$ after standing for 7.5 hours and a gel strength of 5,000 to 9,000 $dyn/cm^2$ after standing for 200 hours, and the gelling base satisfies the following relationship:
Gel strength after standing for 7.5 hours ≤ Gel strength after standing for 200 hours;
the gelling base comprising:

4 parts by mass of glycerol, 4 parts by mass of propylene glycol, 5 parts by mass of a water-soluble acrylate polymer, 0.2 parts by mass of a dried aluminum hydroxide gel exhibiting acid reactivity with 0.1 N-HCl of 180 seconds, 0.25 parts by mass of tartaric acid, and 86.55 parts by mass of distilled water.

Item 2. The water-soluble acrylate polymer according to Item 1, which has a neutralization degree of 42 to 90 mol%.
Item 3. The water-soluble acrylate polymer according to Item 1 or 2, which is obtained by subjecting acrylic acid and a salt thereof to a polymerization reaction to such an extent that its conversion becomes not less than 85 mol%.
Item 4. A gelling base comprising the water-solubie acrylate polymer of any one of Items 1 to 3.
Item 5. A poultice comprising the gelling base of Item 4.
Item 6. A cooling sheet comprising the gelling base of Item 4.

The present invention is explained in detail below.

The water-soluble acrylate polymer of the present invention makes it possible to obtain a gelling base that has the following properties when it comprises the components and the proportions mentioned below.
When the gelling base is gelated by being allowed to stand under the conditions of 25°C and a relative humidity of 60%, the resulting gel has a gel strength of 3,200 to 7,000 $dyn/cm^2$ after standing for 7.5 hours and a gel strength of 5,000 to 9,000 $dyn/cm^2$ after standing for 200 hours, wherein the following relationship is satisfied.
Gel strength after standing for 7.5 hours ≤ Gel strength after standing for 200 hours.
The gelling base comprises 4 parts by mass of glycerol, 4 parts by mass of propylene glycol, 5 parts by mass of a water-soluble acrylate polymer, 0.2 parts by mass of a dried aluminum hydroxide gel exhibiting acid reactivity with 0.1 N-HCl of 180 seconds, 0.25 parts by mass of tartaric acid, and 86.55 parts by mass of distilled water.

Here the expression "gel exhibiting acid reactivity with 0.1 N-HCl of 180 seconds" means that when 0.8 g of dried aluminum hydroxide gel is added to 0.1 N-HCl (50 ml) having a temperature of 37±0.5°C while stirring, it takes 180 seconds for the mixture to have a pH value of 3.0.

The gel obtained by gelating the gelling base comprising the water-soluble acrylate polymer of the present invention at the aforementioned ratio has a gel strength of 3,200 to 7,000 $dyn/cm^2$ and preferably 3,200 to 5,500 $dyn/cm^2$ after being allowed to stand for 7.5 hours under the conditions of 25°C and a relative humidity of 60%.

In the present invention, the "gel strength (strength of the gel)" is the value measured by the following measurement method.

Gel Strength

The gel strength is measured using a curdmeter (produced by I TECHNO Co., Ltd., product name: Curdmeter MAX, model name: ME-303) under the following measurement conditions:

Load: 100 g, diameter of pressure-sensitive shaft: 16 mm, carriage speed: 7 seconds/inch, measurement mode: viscous.

The reason for measuring the gel strength after allowing the gelling base comprising the components described above to stand for 7.5 hours under the conditions of 25 °C and a relative humidity of 60% is to evaluate the cure rate of the gel. When a poultice or the like is produced using a gelling base that contains a water-soluble acrylate polymer, the following method is usually employed. That is, a gel is aged for a predetermined period of time under the conditions of, for example, 25 °C and a relative humidity of 60%. Therefore, the present invention defines the conditions for ageing a gel as the conditions under which the cure rate of the gel is evaluated. The reason for setting the standing time to 7.5 hours is that this allows the gel to adequately cure, reducing the variation in the measurement values of the gel strength. If the standing time is set to 5 hours, the gel would not be sufficiently cured, easily causing variations in the measurement values of the gel strength. However, if the standing time is set to 10 hours, the gel is almost completely cured, and such a gel may not be suitable for evaluating the cure rate.

More specifically, if a gelling base has a gel strength of 3,200 to 7,000 $dyn/cm^2$ after being allowed to stand for 7.5 hours under the conditions of 25°C and a relative humidity of 60%, the gelling base exhibits a desirable rate for curing the gel. When a poultice or the like is produced using a gelling base that contains a water-soluble acrylate polymer, the deformation and seeping of the gelling base and penetration of the gelling base to the back side of the nonwoven fabric can be prevented during aging (while standing still), and the deformation of the resulting poultice and the like can also be prevented by preventing the evaporation of moisture from the cut surface after being cut into a desired size.

When a poultice or the like is produced using a gelling base containing a water-soluble acrylate polymer, in view of the ease of gelling base application, it is preferable that the gelling base have a gel strength of 3,200 to 5,500 dyn/cm$^2$ after being allowed to stand for 7.5 hours under the conditions of 25°C and a relative humidity of 60%. Note that the gelling base is applied immediately after being prepared, and is not aged for a predetermined period of time. However, the gelling base gradually starts curing immediately after it is prepared; therefore, by measuring the gel strength after the gelling base stands for 7.5 hours, the coatability of the gelling base can be evaluated.

A gelling base that contains the water-soluble acrylate polymer of the present invention at the proportions specified above has a gel strength of 5,000 to 9,000 dyn/cm$^2$, and preferably 6,000 to 9,000 dyn/cm$^2$ after being allowed to stand for 200 hours under the conditions of 25°C and a relative humidity of 60% to form a gel.

The reason for measuring the gel strength after allowing the gelling base comprising the components described above to stand for 200 hours under the conditions of 25°C and a relative humidity of 60% is to evaluate the gel strength of a completely cured gel.

More specifically, if the gelling base has a gel strength within the range of 5,000 to 9,000 dyn/cm$^2$ after standing for 200 hours under the conditions of 25°C and a relative humidity of 60%, the gelling base has an adequate hardness (strength). When a poultice or the like is produced using such a water-soluble acrylate polymer-containing gelling base and the resulting poultice or the like is stretched, such a use not only prevents the gel from being permanently stretched, but also allows it to stretch without breaking.

When a poultice or the like is produced using the water-soluble acrylate polymer-containing gelling base, the gel strength after standing for 200 hours under the conditions of 25°C and a relative humidity of 60% is preferably 6,000 to 9,000 dyn/cm$^2$ in order to allow it to be easily restored to its original state when the resulting poultice or the like is stretched and then the stretching force is released.

Regarding the relation between the gel strength after standing for 7.5 hours and that after standing for 200 hours, the gel strength after standing for 200 hours is generally higher; therefore, the formula mentioned below is satisfied. The following relationship must also be satisfied from the viewpoint of gelling base handling ease during the production of a poultice or the like using the water-soluble acrylate polymer-containing gelling base, and rendering the desirable elongation and resilience (stretchability) of the gel in the resulting poultice or the like.

Gel strength after standing for 7.5 hours ≤ Gel strength after standing for 200 hours

The water-soluble acrylate polymer of the present invention is not limited as long as a gelling base comprising the components and the proportions thereof described above has the following properties. The gelling base has a gel strength of 3,200 to 7,000 dyn/cm$^2$ after gelating by being allowed to stand for 7.5 hours and 5,000 to 9,000 dyn/cm$^2$ after being allowed to stand for 200 hours under the conditions of 25°C and a relative humidity of 60%, with the following relationship being satisfied:

Gel strength after standing for 7.5 hours ≤ Gel strength after standing for 200 hours.

Preferable examples of such water-soluble acrylate polymers include those in which the polymerization degree is desirably controlled when polymerizing an acrylic acid or a salt thereof so that extreme low-molecular-weight polymers and extreme high-molecular-weight polymers are not formed. The polymerization method is not particularly limited, and typical polymerization methods such as an inversed phase suspension polymerization method and an aqueous polymerization method can be employed.

The inversed phase suspension polymerization method is explained in detail as one embodiment of the present invention. The inversed phase suspension polymerization method is conducted by, for example, subjecting an acrylic acid and a salt thereof to inversed phase suspension polymerization in a water-in-oil system in a petroleum-based hydrocarbon solvent that contains a surfactant and/or a high-molecular-weight-based dispersant, using a radical polymerization initiator. The inversed phase suspension polymerization method may be conducted in two or more steps, wherein a slurry of a water-soluble acrylate polymer is obtained by inversed phase suspension polymerization and an acrylic acid and a salt thereof are further added to the resulting slurry.

The acrylic acid and a salt thereof are generally used in the form of an aqueous solution. The concentration of the acrylic acid and a salt thereof in the aqueous solution is preferably 15 mass% to a saturated concentration in order to quickly advance the polymerization reaction.

It is preferable that the acrylate be neutralized by using an alkaline neutralizer. The neutralization degree using an alkaline neutralizer is preferably 42 to 90 mol% in order to suitably control the gel strength of the gelling base that comprises the water-soluble acrylate polymer at the ratio described above after it is gelated by being allowed to stand for 200 hours under the conditions of 25°C and a relative humidity of 60%, and to adequately control the elongation when stretching a poultice or like product that is produced using the water-soluble acrylate polymer-containing gelling base. The gel strength thereof is more preferably 47 to 60 mol% to allow easy restoration to the original shape when the resulting poultice or the like is stretched and the stretching force is released. If the neutralization degree obtained by using an alkaline neutralizer is less than 42 mol%, a gel prepared using such a water-soluble acrylate polymer tends to become unduly hard and the gel strength after standing for 200 hours may exceed 9,000 dyn/cm$^2$. If the neutralization degree obtained by using an alkaline neutralizer exceeds 90 mol%, a gel prepared using such a water-soluble acrylate polymer tends to become unduly soft, and the gel strength after standing for 200 hours may become less than 5,000 dyn/cm$^2$.

Specific examples of acrylate include lithium acrylate, sodium acrylate, potassium acrylate, ammonium acrylate, etc. Among these acrylates, sodium acrylate and potassium acrylate are suitably used, and sodium acrylate is particularly suitable for use.

The water-soluble acrylate polymer of the present invention may contain polymerizable monomers other than acrylic acid and a salt thereof to such an extent that it does not hamper the polymerisation reaction, and it does not adversely affect the performance of the resulting water-soluble acrylate polymer.

Examples of radical polymerization initiators include potassium persulfate, ammonium persulfate, sodium persulfate and like persulfates; methyl ethyl ketone peroxide, methyl isobutyl ketone peroxide, di-tert-butyl peroxide, tert-butyl cumyl peroxide, tert-butyl peroxyacetate, tert-butylperoxy isobutyrate, tert-butylperoxy pivalate, hydrogen peroxide and like peroxides; 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis {2-[1-(2-hydroxyethyl)-2-imidazolin-2-yl] propane} dihydrochloride, 2,2'-azobis {2-methyl-N-[1,1-bis(hydroxymethyl)-2-hydroxyethyl]propionamide}, 2,2'-azobis [2-methyl-N-(2-hydroxyethyl)-propionamide], 4,4'-azobis(4-cyanovaleric acid) and like azo compounds; etc. These radical polymerization initiators may be used singly or in combination. Among these radical polymerization initiators, potassium persulfate, ammonium persulfate, sodium persulfate and 2,2'-azobis(2-amidinopropane)dihydrochloride are suitably used as they are easily available from an industrial perspective and have good storage stability.

The amount of the radical polymerization initiator used is preferably 0.015 to 0.15 parts by mass relative to 100 parts by mass of acrylic acid and a salt thereof in order to shorten the polymerization reaction time, prevent a too rapid polymerization reaction, desirably control the degree of polymerization, and control the gel strength of a gel obtained by gelating the gelling base that comprises the resulting water-soluble acrylate polymer at the proportion described above by being allowed to stand for 7.5 hours under the conditions of 25°C and a relative humidity of 60%. Such an amount of the radical polymerization initiator is preferable also in order to prevent the gelling base from being deformed, seeping from the base and penetrating to the back side of the nonwoven fabric while being aged when the water-soluble acrylate polymer-containing gelling base is used to produce a poultice or the like. In addition, when the water-soluble acrylate polymer-containing gelling base is used to produce a poultice or the like, the amount of the radical polymerization initiator used is preferably 0.02 to 0.055 parts by mass relative to 100 parts by mass of acrylic acid and a salt thereof from the viewpoint of easy gelling base application. When the amount of the radical polymerization initiator is less than 0.015 parts by mass, not only the polymerization reaction may be prolonged but also the gel obtained using the resulting water-soluble acrylate polymer tends to become undesirably hard, and this may cause the gel strength after standing for 7.5 hours to exceed 7,000 dyn/cm$^2$. When the amount of the radical polymerization initiator exceeds 0.15 parts by mass, the polymerization reaction proceeds too quickly, resulting in a too rapid reaction. This may make it impossible to control the polymerization reaction and make the cure rate of the gel too slow when a gel is prepared using the resulting water-soluble acrylate polymer. This may cause the gel strength after standing for 7.5 hours to be less than 3,200 dyn/cm$^2$.

The radical polymerization initiator may be used as a redox-polymerization initiator in combination with sodium sulfite, sodium hydrogensulfite, iron (II) sulfite and like sulfites; D-ascorbic acid, L-ascorbic acid, rongalite and like reducing agents; etc.

In the production of the water-soluble acrylate polymer of the present invention, the addition of a water-soluble chain transfer agent is preferable in order to control the degree of polymerization, so that the formation of an extreme low-molecular-weight polymer or an extreme high-molecular-weight polymer can be prevented. Examples of water-soluble chain transfer agents include hypophosphites, phosphorous acid, thiol groups, secondary alcohols, amines, etc. These water-soluble chain transfer agents may be used singly or in combination. Among these water-soluble chain transfer agents, sodium hypophosphite, potassium hypophosphite and like hypophosphites are suitably used because they have no odor and are desirable in terms of sanitary and safety aspects.

In order to suitably control the degree of polymerization, the amount of the water-soluble chain transfer agent is preferably 0.001 to 2 parts by mass, and more preferably 0.001 to 1.7 parts by mass relative to 100 parts by mass of acrylic acid and a salt thereof. When the amount of the water-soluble chain transfer agent is less than 0.001 parts by mass, the effect of the water-soluble chain transfer agent may not be fully exhibited. When the amount of the water-soluble chain transfer agent exceeds 2 parts by mass, the proportion of the low-molecular-weight polymer undesirably increases and the gel cure rate tends to become slow during gel preparation. This may make the gel strength after standing for 7.5 hours less than 3,200 dyn/cm$^2$ and may lower the adhesiveness of the gel when a poultice or the like is produced using the water-soluble acrylate polymer-containing gelling base, because the salt content in the gel used in the poultice or the like increases.

Examples of surfactants include polyglyceryl fatty acid esters, sucrose fatty acids esters, sorbitan fatty acid esters, polyoxyethylenesorbitan fatty acid esters, polyoxyethyleneglycerine fatty acid esters, sorbitol fatty acid esters, polyoxyethylenesorbitol fatty acid esters, polyoxyethylene alkyl ether, polyoxyethylene alkyl phenyl ether, polyoxyethylene castor oil, polyoxyethylene hydrogenated castor oil, alkyl allyl formaldehyde condensed polyoxyethylene ethers, polyoxyethylene polyoxypropylene block copolymers, polyoxyethylene polyoxy propyl alkyl ethers, polyethylene glycol fatty acid esters, polyoxyethylene alkylamine, phosphoric esters of polyoxyethylene alkyl ether, phosphoric esters of polyoxyethylene alkyl aryl ether, etc. These surfactants may be used singly or in combination. Among these surfactants, sorbitan fatty acid esters, polyglyceryl fatty acid esters and sucrose fatty acids esters are suitably used from the viewpoint of the dispersion stability of the aqueous solution of an acrylic acid and a salt thereof.

Examples of high-molecular-weight-based dispersants include maleic anhydride modified polyethylene, maleic anhydride modified polypropylene, maleic anhydride modified ethylene/propylene copolymers, maleic anhydride modified EPDM (ethylene/propylene/diene terpolymers), maleic anhydride modified polybutadiene, ethylene/maleic anhydride copolymers, ethylene/propylene/maleic anhydride copolymers, butadiene/maleic anhydride copolymers, oxidation-type polyethylene, ethylene/acrylic acid copolymers, ethylcellulose, ethylhydroxyethyl cellulose, etc. These high-molecular-weight-based dispersants may be used singly or in combination. Among these, maleic anhydride modified polyethylene, maleic anhydride modified polypropylene, maleic anhydride modified ethylene propylene copolymers, oxidation-type polyethylene, ethylene/acryl acid copolymers are suitably used from the viewpoint of the dispersion stability of the aqueous solution of an acrylic acid and a salt thereof.

The amount of each surfactant and/or high-molecular-weight-based dispersant is preferably 0.1 to 5 parts by mass and more preferably 0.2 to 3 parts by mass relative to 100 parts by mass of acrylic acid and a salt thereof in order to maintain excellent dispersion of an aqueous solution of acrylic acid and a salt thereof in a petroleum-based hydrocarbon solvent, and to obtain a dispersion effect that achieves a good balance with the amount of surfactant and/or high-molecular-weight-based dispersants used. When the amount of each surfactant and/or high-molecular-weight-based dispersant used is less than 0.1 parts by mass, the dispersibility of acrylic acid and a salt thereof becomes undesirably low, and this may result in irregular polymerization. When the amount of the surfactant and/or high-molecular-weight-based dispersant exceeds 5 parts by mass, the dispersion effect that is in good balance with the amount used may not be achieved.

Examples of petroleum-based hydrocarbon solvents include *n*-hexane, *n*-heptane, *n*-octane, ligroin and like aliphatic hydrocarbons; cyclopentane, methylcyclopentane, cyclohexane, methylcyclohexane and like alicyclic hydrocarbons; benzene, toluene, xylene and like aromatic hydrocarbons; etc. These petroleum-based hydrocarbon solvents may be used singly or in combination. Among these petroleum-based hydrocarbon solvents, *n*-hexane, *n*-heptane, and cyclohexane are preferable as they are easily available from an industrial perspective, stable in quality and inexpensive.

The amount of the petroleum-based hydrocarbon solvent is preferably 50 to 600 parts by mass, and more preferably 80 to 550 parts by mass relative to 100 parts by mass of acrylic acid and a salt thereof in order to easily control the polymerization temperature by removing the heat of polymerization.

The reaction temperature in the polymerization varies depending on the radical polymerization initiator used. However, the reaction temperature is preferably 20 to 110°C. Having a reaction temperature within that range facilitates rapid polymerization to shorten the polymerization time, improves productivity, and allows easy removal of the polymerization heat to promote a smooth reaction. The reaction temperature is more preferably 40 to 90°C in order to easily control the polymerization temperature and degree of polymerization. When the reaction temperature is lower than 20°C, the rate of polymerization becomes slow and prolongs the polymerization time, and is thus economically undesirable. When the reaction temperature exceeds 110°C, removal of the polymerization heat becomes difficult. This, in turn, may make the conduction of a smooth reaction difficult.

The polymerization reaction time varies depending on the reaction temperature; however, 10 minutes to 8 hours is preferable in order to desirably control the degree of polymerization by allowing acrylic acid and a salt thereof to sufficiently react. In order to obtain the water-soluble acrylate polymer of the present invention, it is desirable to increase the conversion to preferably not less than 85 mol%, and more preferably not less than 90 mol% by allowing acrylic acid and a salt thereof to sufficiently react for about 10 minutes to 6 hours during the polymerization reaction after the internal temperature reaches its peak. When the conversion is less than 85 mol%, a large amount of unreacted acrylic acid and a salt thereof remains, and the unreacted substance reacts in the drying process after the polymerization to form a low-molecular-weight polymer, thereby increasing the proportion of the low-molecular-weight polymer. This tends to slow the cure rate of the gel when a gel is prepared, and may result in a gel strength of less than 3,200 dyn/cm$^2$ after standing for 7.5 hours. In the present invention, the "conversion" is obtained based on the amount of acrylic acid remaining in the polymerization system after the completion of the polymerization reaction. More specifically, this value is obtained by the measurement method that is described later.

After the polymerization reaction is completed, moisture and the petroleum-based hydrocarbon solvent are removed by heating, for example, at 80 to 200°C, to dry the resulting substance, obtaining a water-soluble acrylate polymer.

An aqueous polymerization method is explained below as one embodiment of the present invention. In the aqueous polymerization method, an acrylic acid and a salt thereof are subjected to aqueous polymerization using a radical polymerization initiator.

In the aqueous polymerization method, the amounts and types of the acrylic acid and a salt thereof, the radical polymerization initiator, and other optional additives are the same as those explained in the inversed phase suspension polymerization method.

In the aqueous polymerization method, the reaction temperature, reaction time, conversion, etc., during the polymerization reaction are the same as those explained in the inversed phase suspension polymerization.

After the polymerization reaction is completed, the resulting gel-like substance is heated, for example, at 80 to 200°C to remove moisture and make it dry, obtaining a water-soluble acrylate polymer.

The present invention also relates to the gelling base that contains the water-soluble acrylate polymer. The gelling base can be prepared by mixing a polyvalent metal salt, a polyhydric alcohol, and a pH adjuster, in addition to the water-soluble acrylate polymer.

A polyvalent metal salt functions as a cross-linking agent to the water-soluble acrylate polymer. Examples of polyvalent metal salts include salts of bivalent to hexavalent metal ions with anions such as chloride ions, sulphate ions, silicate ions and phosphate ions. Specific examples of polyvalent metal ions include aluminum ions, calcium ions, iron ions and the like. Specific examples of polyvalent metal salts include aluminum hydroxide, aluminum sulfate, aluminum silicate, aluminum phosphate, aluminum glycinate, calcium hydroxide, iron (III) sulfate, etc. These polyvalent metal salts may be used singly or in combination.

The polyhydric alcohols function as a water retention agent. Specific examples of polyhydric alcohols include glycerol, polypropylene glycol, sorbitol, butylene glycol, etc. These polyhydric alcohols may be used singly or in combination.

The pH adjuster promotes the elution of metal ions from a polyvalent metal salt, and controls the pH of the gel base. Specific examples of pH adjusters include tartaric acid, lactic acid, citric acid and like organic acids. The pH adjusters may be used singly or in combination.

One example of a method for producing a gel base is mixing a water-soluble acrylate polymer, a polyvalent metal salt, and a polyhydric alcohol to prepare a dispersion liquid. An aqueous solution containing tartaric acid and water is separately prepared. Thereafter, the dispersion liquid and the aqueous solution are mixed to obtain the gel base.

When added to the gelling base, the water-soluble acrylate polymer of the present invention renders an adequate gel strength and achieves a desirable cure rate when reacted with a polyvalent metal. Therefore, an excellent poultice, cooling sheet, etc., can be provided by using this gelling base.

The method for producing the poultice or cooling sheet is not particularly limited. For example, a gelling base comprising the water-soluble acrylate polymer of the present invention and optionally any additives is applied to a support such as nonwoven fabric, and the surface to which the gelling base is applied is covered with a liner such as polyethylene film. The result is cut into a desirable size if necessary, placed in a package, and then cured and aged.

Examples of optional additives for producing a poultice are methyl salicylate, L-menthol, D,L-camphor, d-α-tocopheryl acetate, etc. Examples of optional additives for producing a cooling sheet are paraben, a pigment, a fragrance, etc.

A polyester nonwoven fabric is an example of a nonwoven fabric. Nonwoven fabrics are commercially available, such as a plaster base fabric (produced by Japan Vilene Company, Ltd.).

EFFECT OF THE INVENTION

The gelling base comprising the water-soluble acrylate polymer of the present invention has a particular gel strength. Therefore, when the gelling base is formed into a poultice or a cooling sheet, it not only renders adequate stretchability but also exhibits a desirable reaction rate with a polyvalent metal. This prevents the gel from penetrating to the back side of a support or seeping from the support when the gel is cured. Furthermore, it allows the gelling base to be easily applied to the support.

BEST MODE FOR CARRYING OUT THE INVENTION

The present invention is explained in detail below with reference to Examples and Comparative Examples. However, the scope of the invention is not limited to these Examples.

The conversions in Examples 1 to 6 and Comparative Examples 1 to 3 were obtained by the procedure described below.

The gel strength, stretchability, and coatability of the water-soluble acrylate polymers obtained in Examples 1 to 6 and Comparative Examples 1 to 3 were evaluated by the procedure described below. Table 1 shows the evaluation results.

The appearances of the poultices obtained in Examples 7 to 12 and Comparative Examples 4 to 6 were evaluated by the following procedure. Table 1 shows the evaluation results.

(1) Conversion

The amount of acrylic acid remaining in the polymerization system was measured by a high-performance liquid chromatograph.

Measurement Conditions

The conditions of the high-performance liquid chromatograph used in the present invention for measuring the amount of residual acrylic acid are shown below.

Model: Produced by Shimadzu Corporation (model number: LC-10AD) Column: Produced by Showa Dekko K.K. (model number: Shodex Ionpac KC-811, 8 mm (diameter) $\times$ 300 mm)
Carrier: phosphoric acid aqueous solution (pH = 2.0)
Carrier flow rate: 0.6 ml/min
Column temperature: 45°C
Detector: UV-VIS Detector (produced by Shimadzu Corporation, model number: SPD-10A)
Measured wavelength: 210 nm
Amount of sample injected: 25 $\mu$l.

Preparation of Calibration Curve

The calibration curve was prepared using an acrylic acid aqueous solution with a known concentration based on the peak area of the acrylic acid aqueous solution in the chromatogram.

Measurement of Amount of Residual Acrylic Acid

After the polymerization reaction was completed, a slurry liquid (about 2 g) containing the polymer was sampled from the polymerization system, and the mass thereof was precisely weighed.
To a 500 ml-beaker, 300±0.1 g of a physiological saline solution (a 0.9 mass% sodium chloride aqueous solution; hereunder, the same sodium chloride aqueous solution was used) was weighed, a magnetic stirrer bar (8 mm (diameter) $\times$ 30 mm, without ring) was placed therein, and the beaker was placed on a magnetic stirrer (produced by Iuchi, model number: HS-30D). Subsequently, the rotation of the magnetic stirrer bar was adjusted to 750 rpm, and the bottom of the vortex generated by the magnetic stirrer bar was adjusted so that it was located in the vicinity of the top portion of the magnetic stirrer bar.
Subsequently, the entire quantity of the aforementioned sample was dispersed by being quickly poured between the center portion of the vortex in the beaker and the sidewall of the beaker. Then, one hour after the initiation of stirring, the monomer that remained in the sample was eluted into the physiological saline solution.
After stirring, the water-soluble acrylate polymer solution was filtered through filter paper No. 3, and the filtrate thus obtained was used as a sample. This sample was poured into a high-performance liquid chromatograph to obtain a chromatogram.
Based on the peak area of the resulting chromatogram, and using a calibration curve prepared in advance, (A) the amount of acrylic acid contained in the sample (unit: mass ppm) was calculated. From (A) the amount of acrylic acid contained in the sample, (B) the amount of acrylic acid contained in the sampled slurry liquid, and (C) the amount of acrylic acid remaining in the polymerization system were calculated. From (C) (the amount of acrylic acid remaining in the polymerization system), (D) the conversion was calculated. The concept of this series of calculations to obtain (D) (the conversion) is shown below.

$$(D) \ (mol\%)=100- \{(C)[g] \div the \ amount \ of \ acrylic \ acid \ used \ [g]\}$$

```
(C) (g) = {total amount in the polymerization system [g] ÷ the
amount of slurry liquid [g]} × (B) (g)
```

```
(B) (g) = {300 g + the amount of slurry liquid (g)} × (A) [ppm].
```

(2) Gel Strength

Preparation of Gel

To a mixed solvent of 4 parts by mass of glycerol and 4 parts by mass of propylene glycol, 5 parts by mass of a water-soluble acrylate polymer and 0.2 parts by mass of a dried aluminum hydroxide gel (produced by Kyowa Chemical Industry Co., Ltd.; model number: S-100, acid reactivity: 0.1 N-HCl = 180 seconds) were added and dispersed, giving Liquid A.

Subsequently, 0.25 parts by mass of tartaric acid was added to 86.55 parts by mass of distilled water, giving Liquid B.

The entire quantity of Liquid A was placed in a 500-ml beaker. The entire quantity of Liquid B was added to Liquid A while stirring at 500 rpm using a pitched paddle having a blade diameter of 75 mm, and the mixture was then stirred for 30 seconds, giving a gel.

Aging of Gel

The above prepared gel (115 to 120 g) was placed in a polyethylene container (produced by AS ONE Corporation, product name: Tight Boy TB-2 and subjected to defoaming for 1.5 minutes using a conditioning mixer (produced by Thinky Co., Ltd., product name: Awatori-rentaro MX-201). The resulting gel was placed in a thermo-hygrostat (produced by ESPEC Corp., product name: LHU-113) adjusted to 25°C, relative humidity of 60%, and then allowed to age for 7.5 hours or 200 hours.

Gel Strength

The gel strength after being aged for a predetermined period of time was measured using a curdmeter (produced by I TECHNO Co., Ltd., product name: Curdmeter MAX, model number: ME-303). The measurement conditions were as shown below:

Load: 100 g, diameter of pressure-sensitive shaft: 16 mm, carriage speed: 7 seconds/inch, and measurement mode: viscous.

(3) Stretchability

In the measurement of (2) Gel Strength described above, the gel after aging for 200 hours was cut into a gel sheet having a diameter of 6 cm and thickness of 1 cm. Portions 1 cm away from the edge of the gel sheet were pinched between an index finger and a thumb and pulled slowly, and the extended length was then measured. The length of the gel sheet was measured after the gel was released from the fingers and allowed to stand still for a while. Evaluation was conducted based on the criteria shown below.

A: Extended to 18 cm or more and returned to 7 cm or less.
B: Extended to 18 cm or more and returned to 9 cm or less.
C: Extended to 18 cm or more but did not return to 9 cm or less.
D: Did not extend to 18 cm or more and the gel sheet was broken.

(4) Coatability

A gel was prepared in the same manner as described in (2) Gel Strength. After preparation, the gel was placed on a PET sheet, and coated using an applicator having 10-mm gaps. The thickness of the sheet was measured using a caliper. Evaluation was conducted based on the criteria shown below.

A: Not less than 8.5 mm and not exceeding 10 mm
B: Not less than 7 mm and less than 8.5 mm,
C: Less than 7 mm

(5) Appearance

The resulting poultice was sealed in an aluminum coated-plastic bag (produced by AS ONE Corporation, product name: Alumilamizip), and then aged for 200 hours in a thermo-hygrostat (produced by ESPEC Corp., product name: LHU-113) adjusted to 25°C, and relative humidity of 60%. After aging, the poultice was removed from the aluminum coated-plastic bag. Evaluation was conducted based on the criteria shown below.

A: No penetration to the back side of the nonwoven fabric, and no seeping.
B: No penetration to the back side of the nonwoven fabric, but some seeping.
C: Penetration to the back side of the nonwoven fabric, but no seeping.
D: Penetration to the back side of the nonwoven fabric, and some seeping.

Example 1

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.
An 80 mass% acrylic acid aqueous solution (92 g, 1.02 mol) was then placed in a 500-ml Erlenmeyer flask. While cooling the flask from the outside, a 30 mass% aqueous sodium hydroxide solution (68.1 g, 0.51 mol) was added thereto dropwise to perform 50 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 51.6 g of ion-exchanged water were added thereto, giving a monomer aqueous solution.
The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 60°C water bath for 0.5 hours, and the reaction was continued. The temperature of the internal solution after 0.5 hours was 59°C. The polymerization slurry liquid thus obtained was cooled to 30°C and sampled to measure the conversion. The conversion was 96 mol%.
After the polymerization was completed, the polymerization slurry liquid was heated in a 125°C oil bath. Azeotropic distillation of *n*-heptane and water was used to remove 109 g of water from the system while making the *n*-heptane reflux. Thereafter, the *n*-heptane in the system was removed by distillation to make the system dry, obtaining 89.4 g of a water-soluble acrylate polymer.

Example 2

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.
An 80 mass% acrylic acid aqueous solution (92 g, 1.02 mol) was then placed in a 500-ml Erlenmeyer flask. While cooling the flask from the outside, a 30 mass% aqueous sodium hydroxide solution (68.1 g, 0.51 mol) was added thereto dropwise to perform 50 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 51.6 g of ion-exchanged water were added thereto, giving a monomer aqueous solution.
The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 55°C water bath for 1 hour, and the reaction was continued. The temperature of the internal solution after 1 hour was 53°C. The polymerization slurry liquid thus obtained was cooled to 30°C and sampled to measure the conversion. The conversion was 94 mol%.

After the polymerization was completed, the polymerization slurry liquid was heated in a 125°C oil bath. Azeotropic distillation of *n*-heptane and water was used to remove 109 g of water from the system while making the *n*-heptane reflux. Thereafter, the *n*-heptane in the system was removed by distillation to make the system dry, obtaining 89.8 g of a water-soluble acrylate polymer.

Example 3

An 80 mass% acrylic acid aqueous solution (27 g, 0.3 mol) was placed in a 300-ml Erlenmeyer flask. While cooling the flask from the outside, a 30 mass% aqueous sodium hydroxide solution (20 g, 0.15 mol) was added thereto dropwise to perform 50 mol% neutralization. To the thus neutralized solution, 52.6 g of ion-exchanged water was added and dissolved, giving a monomer aqueous solution.

To a 500-ml five-necked cylindrical round-bottom flask equipped with a reflux condenser, a dropping funnel, a nitrogen introduction tube, a stirrer, and a stirring blade, the entire quantity of this monomer aqueous solution was added. The atmosphere inside the system was replaced with nitrogen, the flask was dipped in a 60°C water bath and then heated to 58°C. 0.54 g of a 2.0 mass% 2,2''-azobis(2-amidinopropane)dihydrochloride aqueous solution as radical polymerization initiators and 0.72 g of a 1.0 mass% sodium hypophosphite monohydrate aqueous solution were added thereto, followed by a polymerization reaction. The system became thicker one minute after the initiation of the polymerization reaction, and stirring was stopped when 2 minutes passed. The system reached the peak temperature (75°C) 4 minutes after the initiation of the polymerization reaction. Thereafter, the flask was placed in a 60°C water bath for 3 hours, and the reaction was continued. The temperature of the internal solution after 3 hours was 58°C. The polymerization liquid thus obtained was cooled to 30°C and sampled to measure the conversion. The conversion was 91 mol%.

After the polymerization was completed, the unified polymer was dried at 120°C for 2 hours. The dried polymer was cracked and dried at 110°C for 2 hours, obtaining 23.4 g of a water-soluble acrylate polymer.

Example 4

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (68.1 g, 0.51 mol) was added thereto dropwise to perform 50 mol% neutralization. To the thus neutralized solution, 0.69 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 51.6 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (76°C) 33 minutes after the initiation of the polymerization reaction. Thereafter, the flask was placed in a 55°C water bath for one hour, and the reaction was continued. The temperature of the internal solution after one hour was 54°C. The polymerization slurry liquid thus obtained was cooled to 30°C and sampled to measure the conversion. The conversion was 90 mol%.

After the polymerization was completed, the polymerization slurry liquid was heated in a 125°C oil bath. Azeotropic distillation of *n*-heptane and water was used to remove 109 g of water from the system while making the *n*-heptane reflux. Thereafter, the *n*-heptane in the system was removed by distillation to make the system dry, obtaining 89.3 g of water-soluble acrylate polymer.

Example 5

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (88.4 g, 0.66 mol) was added thereto dropwise to perform 65 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 39.5 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (76°C) 30 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 91 mol%.

After the polymerization was completed, the polymerization slurry liquid was heated in a 125°C oil bath. Azeotropic distillation of *n*-heptane and water was used to remove 123 g of water from the system while making the *n*-heptane reflux. Thereafter, the *n*-heptane in the system was removed by distillation to make the system dry, obtaining 91.9 g of a water-soluble acrylate polymer.

Example 6

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (64.0 g, 0.48 mol) was added thereto dropwise to perform 47 mol% neutralization. To the thus neutralized solution, 0.69 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 54.5 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (76°C) 33 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 92 mol%.

After the polymerization was completed, the polymerization slurry liquid was heated in a 125°C oil bath. Azeotropic distillation of *n*-heptane and water was used to remove 108 g of water from the system while making the *n*-heptane reflux. Thereafter, the *n*-heptane in the system was removed by distillation to make the system dry, obtaining 86.6 g of a water-soluble acrylate polymer.

Comparative Example 1

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (68.1 g, 0.51 mol) was added thereto dropwise to perform 50 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 51.6 g of ion-exchanged water were added thereto, giving a monomer aqueous solution.

The entire quantity of this monomer aqueous solution was added to the cylindrical round-bottom flask. The atmosphere inside the system was fully replaced with nitrogen. The flask was dipped in a 60°C water bath to heat it, followed by a polymerization reaction. The system reached the peak temperature (79°C) 30 minutes after the initiation of the polymerization reaction. The resulting polymerization slurry liquid was immediately cooled to 30°C and the conversion thereof was measured. The conversion was 78 mol%.

After the polymerization was completed, the polymerization slurry liquid was heated in a 125°C oil bath. Azeotropic distillation of *n*-heptane and water was used to remove 109 g of water from the system while making the *n*-heptane reflux. Thereafter, the *n*-heptane in the system was removed by distillation to make the system dry, obtaining 89.4 g of a water-soluble acrylate polymer.

Comparative Example 2

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.
An 80 mass% acrylic acid aqueous solution (92 g, 1.02 mol) was then placed in a 500-ml Erlenmeyer flask. While cooling the flask from the outside, a 30 mass% aqueous sodium hydroxide solution (88.4 g, 0.66 mol) was added thereto dropwise to perform 65 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 39.5 g of ion-exchanged water were added thereto, giving a monomer aqueous solution.
The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (76°C) 30 minutes after the initiation of the polymerization reaction. The resulting polymerization slurry liquid was immediately cooled to 30°C and sampled to measure the conversion. The conversion was 76 mol%.
After the polymerization was completed, the polymerization slurry liquid was heated in a 125°C oil bath. Azeotropic distillation of *n*-heptane and water was used to remove 123 g of water from the system while making the *n*-heptane reflux. Thereafter, the *n*-heptane in the system was removed by distillation to make the system dry, obtaining 91.7 g of a water-soluble acrylate polymer.

Comparative Example 3

A 1,000-ml five-necked cylindrical round-bottom flask equipped with a reflux condenser, a dropping funnel, a nitrogen introduction tube, a stirrer and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene propylene copolymer (produced by Mitsui Chemicals, Inc., Hi-wax 1105A) were added thereto. The mixture was heated to 80°C while stirring to dissolve the surfactant, and then cooled to 55°C.
An 80 mass% acrylic acid aqueous solution (92 g, 1.02 mol) was then placed in a 500-ml Erlenmeyer flask. While cooling the flask from the outside, a 30 mass% aqueous sodium hydroxide solution (54.5 g, 0.41 mol) was added thereto dropwise to perform 40 mol% neutralization. To the thus neutralized solution, 1.15 g of a 2.0 mass% 2,2'-azobis (2-amidinopropane)dihydrochloride aqueous solution as radical polymerization initiators and 0.92 g of a 1.0 mass% sodium hypophosphite monohydrate aqueous solution, and 51.6 g of ion-exchanged water were added thereto, giving a monomer aqueous solution.
The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 96 mol%.
After the polymerization was completed, the polymerization slurry liquid was heated in a 125°C oil bath. Azeotropic distillation of *n*-heptane and water was used to remove 106 g of water from the system while making the *n*-heptane reflux. Thereafter, the *n*-heptane in the system was removed by distillation to make the system dry, obtaining 86.1 g of a water-soluble acrylate polymer.

Example 7

To a mixed solvent of 4 parts by mass of glycerol and 4 parts by mass of propylene glycol, 5 parts by mass of the water-soluble acrylate polymer obtained in Example 1 and 0.2 parts by mass of a dried aluminum hydroxide gel (produced by Kyowa Chemical Industry Co., Ltd.; model number: S-100, acid reactivity: 0.1 N-HCl = 180 seconds) were added and dispersed, giving Liquid A.
Subsequently, 0.25 parts by mass of tartaric acid was added to 86.55 parts by mass of distilled water, giving Liquid B.

The entire quantity of Liquid A was placed in a 500 ml-beaker. The entire quantity of Liquid B was added to Liquid A while stirring at 500 rpm using a pitched paddle having a blade diameter of 75 mm, and the mixture was then stirred for 30 seconds, giving a gel.

The aforementioned gel was spread over a polyester nonwoven fabric (produced by Japan Vilene Company, Ltd., product name: plaster base fabric) in such a manner that the thickness of the coating became 2 mm per side. The coated surfaces were covered with nylon film. The resulting sheet was cut into a size of 100 mm $\times$ 50 mm, obtaining a poultice.

Example 8

A poultice was prepared in the same manner as in Example 7 except that the water-soluble acrylate polymer obtained in Example 2 was used instead of the water-soluble acrylate polymer obtained in Example 1.

Example 9

A poultice was prepared in the same manner as in Example 7 except that the water-soluble acrylate polymer obtained in Example 3 was used instead of the water-soluble acrylate polymer obtained in Example 1.

Example 10

A poultice was prepared in the same manner as in Example 7 except that the water-soluble acrylate polymer obtained in Example 4 was used instead of the water-soluble acrylate polymer obtained in Example 1.

Example 11

A poultice was prepared in the same manner as in Example 7 except that the water-soluble acrylate polymer obtained in Example 5 was used instead of the water-soluble acrylate polymer obtained in Example 1.

Example 12

A poultice was prepared in the same manner as in Example 7 except that the water-soluble acrylate polymer obtained in Example 6 was used instead of the water-soluble acrylate polymer obtained in Example 1.

Comparative Example 4

A poultice was prepared in the same manner as in Example 7 except that the water-soluble acrylate polymer obtained in Comparative Example 1 was used instead of the water-soluble acrylate polymer obtained in Example 1.

Comparative Example 5

A poultice was prepared in the same manner as in Example 7 except that the water-soluble acrylate polymer obtained in Comparative Example 2 was used instead of the water-soluble acrylate polymer obtained in Example 1.

Comparative Example 6

A poultice was prepared in the same manner as in Example 7 except that the water-soluble acrylate polymer obtained in Comparative Example 3 was used instead of the water-soluble acrylate polymer obtained in Example 1.

Table 1

| Water-Soluble Acrylate Polymer | Gel Strength (dyn/cm$^2$) | | Strechability | Coatability | Poultice | Appearance |
|---|---|---|---|---|---|---|
| | After standing for 7.5 hours | After standing for 200 hours | | | | |
| Example 1 | 3,800 | 7,000 | A | A | Example 7 | A |
| Example 2 | 3,400 | 6,800 | A | A | Example 8 | A |

(continued)

| Water-Soluble Acrylate Polymer | Gel Strength (dyn/cm$^2$) | | Strechability | Coatability | Poultice | Appearance |
|---|---|---|---|---|---|---|
| | After standing for 7.5 hours | After standing for 200 hours | | | | |
| Example 3 | 3,200 | 7,000 | A | A | Example 9 | A |
| Example 4 | 5,700 | 7,000 | A | B | Example 10 | A |
| Example 5 | 3,200 | 5,200 | B | A | Example 11 | A |
| Example 6 | 6,700 | 8,800 | A | B | Example 12 | A |
| Comp.Ex.1 | 1,500 | 6,800 | A | A | Comp.Ex.4 | D |
| Comp.Ex.2 | 1,100 | 4,700 | C | A | Comp.Ex.5 | D |
| Comp.Ex.3 | 5,300 | 12,000 | D | A | Comp.Ex.6 | A |

As is clear from Table 1, the water-soluble acrylate polymers of Examples 1 to 6 are superior to the water-soluble acrylate polymers of Comparative Examples 1 to 3 in terms of stretchability and coatability. When a gelling base containing a specific amount of the water-soluble acrylate polymer of any one of Examples 1 to 6 is gelated by being allowed to stand under the conditions of 25 °C and a relative humidity of 60%, the resulting gel has a gel strength of 3,200 to 7,000 dyn/cm$^2$ after standing for 7.5 hours and a gel strength of 5,000 to 9,000 dyn/cm$^2$ after standing for 200 hours, wherein the following relationship is satisfied.

Gel strength after standing for 7.5 hours ≤ Gel strength after standing for 200 hours.

Furthermore, the poultices of the present invention obtained in Examples 7 to 12 were superior to those of Comparative Examples 4 to 6 in terms of their appearance because they were free from gel penetration to the back surface of the nonwoven fabric and gel seeping from the support.

INDUSTRIAL APPLICABILITY

The gelling base prepared using the water-soluble acrylate polymer of the present invention has a specific gel strength; therefore, when formed into a poultice or a cooling sheet, the resulting product has not only a desirable stretchability, but also a preferable reaction rate with a polyvalent metal. This prevents gel penetration to the back side of a support or seeping of the gel from the support when the gel is cured. Furthermore, the gelling base of the present invention can be easily coated on the surface of the support.

**Claims**

1. A water-soluble acrylate polymer that makes it possible to obtain a gelling base having a gel strength recited below when the gelling base comprises the components and the proportions thereof listed below;
   when the gelling base is gelated by being allowed to stand under the conditions of 25°C and a relative humidity of 60%, the resulting gel has a gel strength of 3,200 to 7,000 dyn/cm$^2$ after standing for 7.5 hours and a gel strength of 5,000 to 9,000 dyn/cm$^2$ after standing for 200 hours, and the gelling base satisfies the following relationship:

   Gel strength after standing for 7.5 hours ≤ Gel strength after standing for 200 hours;
   the gelling base comprising:

   4 parts by mass of glycerol, 4 parts by mass of propylene glycol, 5 parts by mass of a water-soluble acrylate polymer, 0.2 parts by mass of a dried aluminum hydroxide gel exhibiting acid reactivity with 0.1 N-HCl of 180 seconds, 0.25 parts by mass of tartaric acid, and 86.55 parts by mass of distilled water.

2. The water-soluble acrylate polymer according to claim 1, which has a neutralization degree of 42 to 90 mol%.

3. The water-soluble acrylate polymer according to claim 1 or 2, which is obtained by subjecting acrylic acid and a salt thereof to a polymerization reaction to such an extent that its conversion becomes not less than 85 mol%.

4. A gelling base comprising the water-soluble acrylate polymer of any one of claims 1 to 3.

**5.** A poultice comprising the gelling base of claim 4.

**6.** A cooling sheet comprising the gelling base of claim 4.

## EP 2 301 976 A1

| | INTERNATIONAL SEARCH REPORT | International application No. |
|---|---|---|
| | | PCT/JP2009/060330 |

A. CLASSIFICATION OF SUBJECT MATTER
C08F20/06(2006.01)i, A61F7/02(2006.01)i, A61F7/10(2006.01)i, A61K8/02
(2006.01)i, A61K8/81(2006.01)i, A61K9/70(2006.01)i, A61K47/02(2006.01)i,
A61K47/10(2006.01)i, A61K47/32(2006.01)i, A61Q19/00(2006.01)i,
According to International Patent Classification (IPC) or to both national classification and IPC

B. FIELDS SEARCHED

Minimum documentation searched (classification system followed by classification symbols)
C08F20/06, A61F7/02, A61F7/10, A61K8/02, A61K8/81, A61K9/70, A61K47/02,
A61K47/10, A61K47/32, A61Q19/00, C09K5/08

Documentation searched other than minimum documentation to the extent that such documents are included in the fields searched
| Jitsuyo Shinan Koho | 1922–1996 | Jitsuyo Shinan Toroku Koho | 1996–2009 |
| Kokai Jitsuyo Shinan Koho | 1971–2009 | Toroku Jitsuyo Shinan Koho | 1994–2009 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-320939 A (Toagosei Co., Ltd.),<br>13 December, 2007 (13.12.07),<br>Claims; Par. No. [0035]; example 2<br>(Family: none) | 1-5<br>6 |
| X | JP 2002-69104 A (Toagosei Co., Ltd.),<br>08 March, 2002 (08.03.02),<br>Claims; example 4<br>(Family: none) | 1-3 |
| Y | JP 2007-297437 A (Toagosei Co., Ltd.),<br>15 November, 2007 (15.11.07),<br>Claims; Par. Nos. [0002], [0011]<br>(Family: none) | 6 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>20 August, 2009 (20.08.09) | Date of mailing of the international search report<br>01 September, 2009 (01.09.09) |
|---|---|
| Name and mailing address of the ISA/<br>Japanese Patent Office | Authorized officer |
| Facsimile No. | Telephone No. |

Form PCT/ISA/210 (second sheet) (April 2007)

| **INTERNATIONAL SEARCH REPORT** | International application No. |
|---|---|
| | PCT/JP2009/060330 |

Continuation of A. CLASSIFICATION OF SUBJECT MATTER
  (International Patent Classification (IPC))

C09K5/08(2006.01)i

              (According to International Patent Classification (IPC) or to both national
              classification and IPC)

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007320939 A